# EUROPEAN PATENT SPECIFICATION

(11) **EP 3 735 218 B1**
(45) Date of publication and mention of the grant of the patent: **23.02.2022**
(21) Application number: 18833333.0
(22) Date of filing: 18.12.2018
(51) Int. Cl.: C07F 1/10, A61K 9/06, A61K 31/192, A61K 31/728, A61K 33/38, A61P 1/02, A61P 31/04, A61P 31/10, A61P 17/02, A61P 41/00, A61P 19/02, A61K 9/00, A61K 47/38, C07F 1/00

(54) **COORDINATION COMPLEXES HAVING MICROBIAL ACTIVITY AND INCORPORABLE IN HYALURONIC ACID COMPOSITIONS**
KOORDINATIONSKOMPLEXE MIT MIKROBIELLER AKTIVITÄT UND IN HYALURONSÄUREZUSAMMENSETZUNGEN INTEGRIERBAR
COMPLEXES DE COORDINATION AYANT UNE ACTIVITÉ MICROBIENNE ET POUVANT ÊTRE INCORPORÉS DANS DES COMPOSITIONS D'ACIDE HYALURONIQUE

(30) Priority: 05.01.2018 IT 201800000576
(43) Date of publication of application: 11.11.2020
(73) Proprietor: Lab Srl, 40123 Bologna (IT)
(72) Inventor: BIGNOZZI, Carlo Alberto, 44124 Ferrara (IT); CARINCI, Francesco, 40124 Bologna (IT); LAURITANO, Dorina, 24121 Bergamo (IT)
(74) Representative: Crugnola, Pietro
(86) International application number: PCT/IB2018/060248
(87) International publication number: WO 2019/135136

(56) References cited:
- EP-A1- 0 727 427
- WO-A1-2017/061878
- US-A1- 2014 271 881

## Description

### Field of the invention

The invention relates to coordination complexes having antimicrobial activity and incorporable in hyaluronic acid compositions, as well as hyaluronic acid compositions incorporating the aforesaid complexes and usable as antimicrobial compositions, in particular in odontostomatology, orthopaedics, cosmetic surgery and in general for promoting the healing of wounds.

### Prior art

The hyaluronic acid (HA) is a linear polysaccharide belonging to the class of the glycosaminoglycans and composed of repeated saccharide units, each of which consists in turn of glucuronic acid and N-acetylglucosamine. The hyaluronic acid is commonly used in the form of physiologically and pharmaceutically acceptable salt, in particular in the form of sodium salt (sodium hyaluronate). The sodium hyaluronate has the following structural formula:

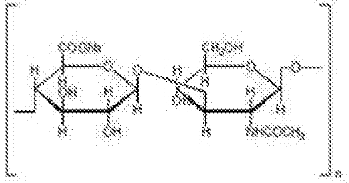

Unlike the other glycosaminoglycans, such as dermatan sulphate or keratan sulphate, the hyaluronic acid does not contain sulphur. The hyaluronic acid is one of the main chemical components of the extracellular matrix of the tissues, inside which the cells and the fibrous constituents of the matrix, such as collagen and elastin, are immersed.

One specific property of the hyaluronic acid is its great capacity to bind water molecules. In the aqueous solutions the hyaluronic acid adopts a flexible and spiralized configuration, which is able to contain and retain a quantity of water equal to about 1000 times the weight of the polymer as sampled (Biopolymers 1970; 9:799-810). This property enables the hyaluronic acid to contribute widely to the maintenance of the integrity of the space and of the extracellular matrix, as well as to control tissue hydration.

Moreover, the hyaluronic acid seems to play a crucial role in the tissue regeneration, since recent studies suggest that the integrity and the balance of the components of the extracellular matrix, which are degraded and reconstructed, ensure a normal function of the tissues and contribute to adjust the process of healing wounds (Skin Pharmacol Physiol. 2004;17:207-13).

The hyaluronic acid has received great attention since its discovery (J. Biol. Chem. 1934, 107, 629-634), inasmuch as it is a versatile and highly functional biopolymer (BioDrugs 2012, 26, 257-268; Carbohydr. Polym. 2013, 92, 1262 - 1279.2 - 6). The hyaluronic acid has been used for different clinical applications, for example in ophthalmology as drug-releasing system (Optom. Vis. Sci. 2014, 91, 32-38), in orthopaedics for the treatment of osteoarthritis by viscosupplementation (Eur. Rev. Med. Pharmacol. Sci. 2014, 18, 3326-3338) and in cosmetic surgery as facial filler (Facial Plast. Surg. 2014, 30, 81-83..16,17).

The hyaluronic acid performs numerous roles in the initial stages of the inflammation, such as providing a structural scaffold through the interaction with the fibrin clot, which modulates the cellular inflammatory infiltration of the extracellular matrix in the inflammatory site. In particular, for the hyaluronic acid a role has been suggested in the migration and adhesion of polymorphonuclear leukocytes and macrophages at the inflammatory site, as well as in the phagocytosis and killing of invading microbes (J Clin Invest. 1980; 66:298-305).

The above-mentioned properties would enable the use of the hyaluronic acid to oppose the colonization and proliferation of pathogenic anaerobic bacteria in the gingival pocket and in the adjacent periodontal tissues and, consequently, to treat periodontal disease effectively. The latter is caused by a bacterial infection that induces an inflammatory response with progressive destruction of the periodontal tissues and, finally, loss of the tooth. The oral microbiota is made of a large number of bacterial species that are able to form a biofilm. The biofilm comprises both pathogenic and saprophytic bacterial species.

The periodontal diseases affect about half of the adult population throughout the world (Lang N., Feres M., Corbet E., Ding Y., Emingil G., Faveri M., Humagain M., Izumi Y., Kamil W., Kemal Y., Mahanonda R., Rajpal J., Sakellari D., Tan W.C., Yamazaki K. Group B. Consensus paper. Non-surgical periodontal therapy: mechanical debridement, antimicrobial agents and other modalities. J Int Acad Periodontal. 2015 Jan; 17(1 Suppl):34-6.). Smoking, alcohol consumption, systemic diseases such as diabetes and osteoporosis, malnutrition and stress are considered additional risk factors. The therapy of the periodontal disease is important to reduce the local inflammation and the bacteraemias. It has been recently stated that the periodontal diseases seem to increase the risk of cardiovascular and pulmonary diseases, the premature birth and the low birth weight (J Int Acad Periodontol.2015 Jan; 17(1 Suppl): 21-30).

The hyaluronic acid has been identified in all periodontal tissues, being particularly considerable in the non-mineralized tissues, such as gum and periodontal ligament, and is present only in small quantity in the mineralized tissues, such as cement and alveolar bone. Recent studies about regenerative surgical procedures (used to treat the periodontal disease) indicate that the reduction of the bacterial load in the lesion site can improve the result of the regenerative therapy. The most common chemotherapeutic agents or drugs for the periodontal diseases are the antimicrobial and anti-inflammatory agents, which can be administered locally or systemically. The known antimicrobial agents for topical use comprise chlorhexidine, tetracyclines and metronidazole (J Clin Periodontol. 2014 Apr; 41 Suppl 15:S1-5).

The hyaluronic acid is a recent addition to the aforesaid local chemotherapeutic agents, because this polysaccharide has shown a certain number of therapeutic properties. In the case of serious periodontal diseases, however, it would be advantageous to increase the therapeutic efficacy of the hyaluronic acid compositions by incorporating antimicrobial agents therein, in particular antiseptics. The antiseptics differ from the antibiotics by the fact that they have a wide spectrum activity and thus can be effective against many types of microorganisms, comprising aerobic and anaerobic bacteria, yeasts, fungi and moulds. The bacterial resistance to the antibiotics is extensively documented in the medical literature, whilst the resistance to the antiseptics has been studied only more recently. The results have suggested that bacteria have developed resistance to the antibiotics, while no sign of resistance has been observed against known antiseptics, such as for example chlorhexidine or silver complexes.

Nevertheless, one drawback connected to the chemical nature of the hyaluronic acid makes substantially difficult to combine stably the hyaluronic acid with antiseptics. In fact, the complex polymeric structure of the hyaluronic acid and the presence of negative charges (in the anionic form of the polysaccharide) cause an almost immediate precipitation and a consequent loss of efficacy of hydrosoluble and positive charged antibacterial agents, such as chlorhexidine, octenidine, benzalkonium salts or other quaternary ammonium salts that are used in the oral treatment of the periodontal diseases.

It should be noted that the drawback disclosed above substantially invalidate also a possible use of the hyaluronic acid in an antimicrobial function for other clinical applications, such as for example cosmetic surgery and orthopaedics. In cosmetic surgery there is in fact the need for hyaluronic acid-based fillers (for example facial fillers) that are provided with antimicrobial properties, since the use of the known fillers may be associated with bacterial, viral or fungal infections that are caused by the loss of integrity of the cutaneous surface (Journal of Dermatology & Dermatologic Surgery 20 (2016) 100-106).

Similarly, in orthopaedics a substantial need is felt for compositions containing hyaluronic acid, for example usable for the viscosupplementation, which are provided with antimicrobial properties. Nevertheless, as previously pointed out, the possible combination of the hyaluronic acid with the known antibacterial agents causes the precipitation of the antibacterial agents, with consequent loss of efficacy.

Another drawback is referable to the chemical nature of the antiseptics that could possibly be combined with the hyaluronic acid, such as for example the silver. In fact, the silver complexes are very often photochemically unstable and scarcely water-soluble.

It should be noted that the silver is a particularly interesting metal from the medical point of view, since this metal displays an antimicrobial activity (the ionic silver is active against over 600 different microbial species) that has been scientifically confirmed in the past (Prog. Med. Chem. 1994). The mechanism of the antimicrobial action of the silver ions (Ag⁺ or Ag (I)) is closely connected to their interaction with the thiolic groups of enzymes and proteins, although other cell components may be involved. The virucidal properties of the silver ions could also be due to a bond with the hydrosulphide groups (Crit. Rev. Environ. Control 1989, 18:295-315.), as well as to the preferential interaction between the silver ions and the DNA bases (Chem. Rev. 1971, 71:439-471). Since the 1990s the silver has regained credibility as therapeutic agent and in particular as an alternative therapeutic agent to antibiotics. The latter are often ineffective due to bacterial resistance phenomena, which phenomena are on the other hand substantially absent for the silver (Adv. Drug Deliv. Rev. 65 (2013) 1803-1815.). The bacterial resistance to the silver is in fact a very rare and often transitory event (Microbiol. Rev. 27 (2003) 341-353; J. Hosp. Infect. 60 (2005) 1-7).

The toxicity of the silver for the bacterial cells has been recognized and documented for some time, whilst the toxicity of the silver for the humans seems to be rather low. The silver does not have a known biological role in the human organism, but it has been traced in the human tissue because of the bioaccumulation with an average concentration of a few µg / kg of moist tissue (Regul. Toxicol. Pharmacol. 68 (2014) 1-7.), this indicating that the human body can tolerate the presence of silver at low doses without toxic effects, very probably through the formation of the silver chloride salt, which has poor solubility in an aqueous medium (Toxicol. In Vitro 27 (2013) 739-744.).

The low toxicity of the silver for the human organism is one of the greater advantages over the other metals that are significant from the medical point of view and the antimicrobial power of the silver ion compounds could be exploited in the medical practice in reasonably safe manner. It has been shown that the silver complexes possess anticancer properties and this has given rise to a new, important research for an alternative to platinum-based complexes for the treatment of tumours (Cancer Lett. 248 (2007) 321-331. J. Inorg. Biochem. 102 (2008) 303-310. Chem. Rev. 109 (2009) 3859-3884. Dalton Trans. (2009) 6894-6902, J. Med. Chem. 53 (2010) 8608-8618).

In recent years, various bacterial and fungal strains provided with multi-resistance to known antimicrobial agents have become a public health problem worldwide (World Health Organization, Antimicrobial Resistance: Global Report on Surveillance, 2014), justifying the use of more powerful antimicrobial compositions for topical use containing metals. In particular, given the proven antimicrobial activity of the silver ions, metal-organic compounds (for example, coordination complexes) incorporating ionic silver could constitute a promising class of compounds suitable for manufacturing "tailored" antimicrobial agents and materials.

Nevertheless, despite the increasing exploitation of the silver coordination complexes as effective antimicrobial agents, the practical application of the latter is still limited, mainly due to the difficulty of preparing hydrosoluble compositions incorporating silver that are thermally and photochemically stable in solution. Therefore, as the need is felt for compositions containing hyaluronic acid and able to incorporate antimicrobial agents, in particular antiseptics, without reducing the effectiveness thereof, the need is similarly felt for metal-organic compounds containing ionic silver, which are thermally and photochemically stable in solution, and are compatible with the anionic form of the hyaluronic acid.

### Objects of the invention

An object of the invention is to improve the known compositions containing hyaluronic acid.

Another object is to make available compositions that contain hyaluronic acid and are provided with a high antimicrobial activity.

Another object is to make available compositions that contain hyaluronic acid and are able to incorporate antimicrobial agents, in particular antiseptics, without causing the precipitation thereof and the consequent loss of efficacy.

Yet another object is to make available compositions containing hyaluronic acid, which are provided with a great antimicrobial activity and are effectively usable in various therapeutic applications, such as treatment of periodontal diseases, cosmetic surgery, orthopaedics and acceleration of the healing of wounds.

Yet another object is to make available compositions containing hyaluronic acid and incorporating an antiseptic agent, in particular a silver-based antiseptic agent, which is thermally and photochemically stable in a solution.

### Brief description of the invention

In a first aspect of the invention, a bi-coordinated silver (I) complex is provided having antimicrobial activity, as defined in claim 1.

In a second aspect of the invention a hyaluronic acid composition is provided containing a mono-coordinated silver (I) complex having antimicrobial activity, as defined in claim 3.

Owing to the invention, it is possible to make hyaluronic acid compositions that enable the previously mentioned objects to be achieved.

In fact, the silver complexes according to the invention are provided with a suitable thermal and photochemical stability in aqueous solution and exhibit a significant antimicrobial activity. The silver complexes according to the invention can be easily incorporated in a hyaluronic acid composition, for example a sodium hyaluronate gel, optionally containing other components (excipients), such as amino acids and xylitol. Unlike the known antiseptics, such as for example the chlorhexidine, the complexes according to the invention do not precipitate when placed in contact with the polymeric structure and the negative charges of the hyaluronate anion and thus do not lose their antimicrobial activity. In this manner, by combining the properties of the hyaluronic acid with the stable antimicrobial activity of the complexes, a hyaluronic acid-based composition is obtained that can be used effectively as an adjuvant for promoting the healing of wounds, for the treatment of periodontal diseases, as antimicrobial filler for cosmetic surgery or for intra-articular orthopaedic treatments (for example viscosupplementation).

### Brief description of the drawings

The invention can be better understood and implemented with reference to the attached drawings that illustrate an embodiment thereof by way of non-limiting example, in which:
Figure 1 is a photograph of two Petri dishes, showing bacterial growth inhibition halos observed after depositing of a sample of an aqueous solution containing the mono-coordinated silver complex according to the invention;
Figure 2 is a photograph of two Petri dishes, showing bacterial growth inhibition halos observed after depositing of a sample of a control gel (dish on left) and a sample of a gel containing hyaluronate and the mono-coordinated silver complex according to the invention (dish on right);
Figure 3 is a photograph of other two Petri dishes, showing bacterial growth inhibition halos observed after depositing of a sample of a control gel (dish on left) and a sample of a gel containing hyaluronate and the mono-coordinated silver complex according to the invention (dish on right);
Figures 4a to 4f are photographs of six Petri dishes, showing bacterial growth inhibition halos observed after depositing of samples of a control gel (Figure 4a; Figure 4d) and samples of gel containing hyaluronate and the mono-coordinated silver complex according to the invention (Figure 4b; Figure 4c; Figure 4e; Figure 4f);
Figures 5a to 5d are photographs of four Petri dishes, showing bacterial growth inhibition halos observed after depositing of corresponding samples of gel containing hyaluronate and the mono-coordinated silver complex according to the invention;
Figure 6 is a photograph of a Petri dish, showing a bacterial growth inhibition halo observed after depositing of a corresponding sample of gel containing hyaluronate and the bi-coordinated silver complex according to the invention.

### Detailed description of the invention

During their research activity, the Inventors have discovered mono-coordinated and bi-coordinated silver (I) complexes that enable a surprisingly new and unexpected technical effect to be obtained. As experimentally verified by the Inventors, the aforesaid complexes are provided with suitable thermal and photochemical stability in aqueous solution and, in particular, do not precipitate in the presence of sodium hyaluronate. More exactly, the complexes according to the invention enable sodium hyaluronate solutions or suspensions to be obtained that are stable in water. Consequently, the complexes according to the invention are incorporable easily and stably (without formation of precipitate) in a hyaluronic acid-based composition, enabling the antimicrobial power of the latter to be increased owing to the presence of an antiseptic (silver) of proven efficacy.

The mono-coordinated silver (I) complexes according to the invention have the general formula:

Na⁺[Ag⁺(L²⁻)]

in which L is a ligand selected from the group consisting of: 4-mercaptobenzoic acid (CAS: 1074-36-8), 3-mercaptobenzoic acid (CAS: 4869-59-4), 2-mercaptobenzoic acid or thiosalicylic acid (CAS: 147-93-3) and 4-mercaptophenylacetic acid (CAS: 39161-84-7). In the aforesaid general formula, the symbol L²⁻ indicates the deprotonated form of the ligand L, in which both the proton of the thiolic group -SH and the proton of the carboxylic group -COOH are dissociated, i.e. the 4-mercaptobenzoate, 3-mercaptobenzoate, 2-mercaptobenzoate and 4-mercaptophenylacetate anions.

The structural formulas of the four aforementioned ligands are shown below:

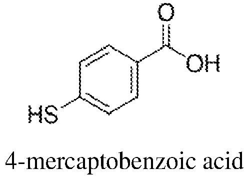

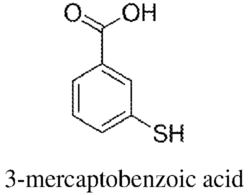

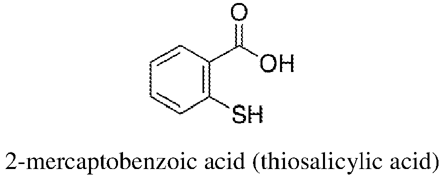

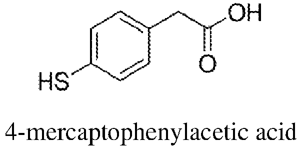

As observed experimentally by the Inventors, only the embodiments of the invention in which the deprotonated form of the ligand L (L²⁻) is the 2-mercaptobenzoate anion produced clear solutions in the presence of sodium hyaluronate. This is probably due to a phenomenon of molecular association (formation of complex molecular species by the combination of two or more molecules of the same nature) among the complexes containing 3-mercaptobenzoate, 4-mercaptobenzoate or 4-mercaptophenylacetate. Nevertheless, as verified experimentally by the Inventors, the phenomenon disclosed above does not influence the antimicrobial activity of the corresponding suspensions and thus does not prevent the embodiments of the invention, in which L²⁻ is 3-mercaptobenzoate, 4-mercaptobenzoate or 4-mercaptophenylacetate, from attaining the objects of the invention.

The bi-coordinated silver (I) complexes according to the invention have the general formula:

Na⁺₂[Ag⁺(L²⁻)(M²⁻)]

in which L is a ligand consisting of 2-mercaptobenzoic acid or thiosalicylic acid (CAS: 147-93-3) and M is a further ligand, different from the ligand L and consisting of 2-hydroxybenzoic acid or salicylic acid (CAS: 69-72-7).

In the aforesaid general formula:
- the symbol L²⁻ indicates the deprotonated form of the ligand L, in which both the proton of the thiolic group -SH and the proton of the carboxylic group -COOH are dissociated, namely the 2-mercaptobenzoate anion;
- the symbol M²⁻ indicates the deprotonated form of the ligand M, in which both the proton of the hydroxyl group -OH and the proton of the carboxylic group -COOH are dissociated, namely the 2-hydroxybenzoate anion.

The structural formula of the 2-hydroxybenzoic acid or salicylic acid (ligand M) is shown below:

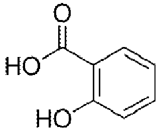

The Inventors verified experimentally that also the complex Na₂⁺[Ag⁺-2-mercaptobenzoate²⁻-2-hydroxybenzoate²⁻] can be incorporated stably into a hyaluronic acid-based composition, in particular a hyaluronic acid gel, providing excellent results in terms of antimicrobial activity.

In the context of the present description, as well as of the appended claims:
- the term "silver ion" and the corresponding chemical symbols "Ag⁺" or "Ag(I)" are used interchangeably;
- the term "2-mercaptobenzoic acid" and the term "thiosalicylic acid" are considered to be synonyms and can be used interchangeably;
- the term "2-hydroxybenzoic acid" and the term "salicylic acid" are considered to be synonyms and can be used interchangeably;
- the term "microorganism" and the term "microbe" are considered to be synonyms and can be used interchangeably;
- the term "antimicrobial" defines the capacity of a substance, or mixture of substances, to kill microorganisms in general and/or to prevent the development thereof;
- the term "antibacterial" defines the capacity of a substance, or mixture of substances, to kill specifically bacteria (bactericide action) and/or to prevent specifically the development of bacteria (bacteriostatic action).

By way of non-limiting example of the invention, the following are disclosed below: a procedure for preparing the complex Na⁺[Ag⁺-2-mercaptobenzoate²⁻] (Example 1); a procedure for preparing the complex Na⁺[Ag⁺-4-mercaptophenylacetate²⁻] (Example 2); a procedure for preparing silver-hyaluronate gel (Example 3); a procedure for preparing a control hyaluronate gel (Example 4); an experimental evaluation of the antimicrobial activity of the complex Na⁺[Ag⁺-2-mercaptobenzoate²⁻] in aqueous solution (Example 5); an experimental evaluation of the antimicrobial activity of the silver-hyaluronate gel (Example 6); an experimental evaluation of the antimicrobial activity of the silver-hyaluronate gel against antibiotic-resistant bacteria (Example 7); an experimental evaluation of the antimicrobial activity *in vivo* of the silver-hyaluronate gel (Example 8); a procedure for preparing the complex Na⁺2[Ag⁺-2-mercaptobenzoate²⁻-2-hydroxybenzoate²⁻] (Example 9); a procedure for preparing silver-hyaluronate gel containing the complex Na⁺2[Ag⁺-2-mercaptobenzoate²⁻-2-hydroxybenzoate²⁻] (Example 10); an experimental evaluation of the antimicrobial activity of the silver-hyaluronate gel containing the complex Na⁺2 [Ag⁺-2-mercaptobenzoate²⁻-2-hydroxybenzoate²⁻] (Example 11).

### Example 1 - Preparation of the complex Na⁺rAg⁺-2-mercaptobenzoate²⁻1

0.14 g of 2-mercaptobenzoic acid (Aldrich, CAS: 4869-59-4; molecular weight: 154.19) have been suspended in 47.89 g of distilled water and 1.82 ml of NaOH 1M (corresponding to two equivalents) have been added to solubilize the ligand, neutralizing the two protons bonded to the sulphur and to the carboxyl function. After complete dissolution of the ligand, which dissolution has occurred in a time comprised between 20 and 30 min, the pH has been measured and adjusted to 7. 0.15 g of AgNO₃ (Aldrich, CAS: 7761-88-8; molecular weight: 169.87) have been added, corresponding to a stechiometric ratio of 1:1 with the ligand. The reaction is almost instantaneous and leads to the formation of an anionic complex, in which the Ag+ ion is bonded to the negative sulphurous component of the 2-mercaptobenzoate. The ionic silver content of the so obtained solution is 0.19 % in weight. The solution has been concentrated in rotating evaporator until precipitation of the complex and the latter has been separated by filtration and dried at 60°C. The (separated and dried) complex has been characterized by elemental analysis. For the elemental analysis of carbon, hydrogen and sulphur, a LECO CHNS analyzer has been used. The silver has been determined with an Optima 3100 XL (Perkin Elmer) optical plasma atomic emission spectrophotometer with torch in axial position, Gem Cone Low Flow nebulizer, nebulization chamber of cylonic type and equipped with an autosampler AS91 Tray F (Perkin Elmer). Calculated percentages of the elements for NaAgC₇H₄O₂S: C, 29.71%; H, 1.42%; S, 11.33; Ag 38.11. Found percentages of the elements for NaAgC₇H₄O₂S: C, 31.89%; H, 1.50%; S, 12.02; Ag, 40.77. An IR spectroscopic analysis has been performed (through Bruker IFS 88S FT-IR spectrophotometer). The analysis has shown that the S-H stretching (i.e., the stretching of the S-H bond) to 930 cm⁻¹ of the 2-mercaptobenzoic acid was absent, which confirms the coordination of the silver to the sulphur.

Through the same synthesis procedure disclosed above, aqueous solutions have been prepared of the two isomeric complexes: Na⁺[Ag⁺-3-mercaptobenzoate²⁻] and Na⁺[Ag⁺-4-mercaptobenzoate²⁻].

### Example 2 - Preparation of the complex Na⁺[Ag⁺-4-mercaptophenylacetate²⁻]

0.12 g of 4-mercaptophenylacetic acid (Aldrich, CAS: 39161-84-7; molecular weight: 168.21) has been suspended in 48.33 g of distilled water and 1.43 ml of NaOH 1M (corresponding to two equivalents) have been added to solubilize the ligand, neutralizing the two protons bonded to the sulphur and to the carboxyl function. After complete dissolution of the ligand, which dissolution has occurred in a time comprised between 20 and 30 min, the pH has been measured and adjusted to 7. 0.12 g of AgNO₃ (Aldrich, CAS: 7761-88-8; molecular weight: 169.87) have been added, corresponding to a 1:1 stechiometric ratio with the ligand. The reaction is almost instantaneous and leads to the formation of an anionic complex, in which the Ag+ ion is bonded to the negative sulphurous component of the 4-mercaptophenylacetate. The ionic silver content of the so obtained solution is 0.19 % in weight.

The solution has been concentrated in rotating evaporator until precipitation of the complex and the complex has been separated by filtration and dried at 60°C. The (separated and dried) complex has been characterized by elemental analysis, which has been performed by using the same apparatuses disclosed in the Example 1. Calculated percentages of the elements for NaAgC₈H₆O₂S: C, 32.35%; H, 2.04%; S, 10.79; Ag, 36.31. Found percentages of the elements for NaAgC₈H₆O₂S: C, 34.70%; H, 2.16%; S, 11.03; Ag, 39.02.

### Example 3 - Preparation of silver-hyaluronate gel

The procedure for the preparation of a hyaluronic acid-based composition according to the invention, namely a hyaluronate gel containing a complex of silver and 2-mercaptobenzoate is disclosed below. This procedure has also been followed for the preparation of hyaluronate gels containing Na⁺[Ag⁺-3-mercaptobenzoate²⁻], Na⁺[Ag⁺⁻4-mercaptobenzoate²⁻] or Na⁺[Ag⁺-4-mercaptophenylacetate²-].

0.3 g of 2-mercaptobenzoic acid have been suspended in 265.57 g of distilled water and 38.9 g of NaOH 0.1M have been added. After complete dissolution of the ligand, the pH has been adjusted to 7 and 0.33 g of AgNO₃ have been added. The so obtained solution has been kept under stirring for 30 min and then sodium hyaluronate (3.0 g), L-proline (0.3g), isoleucine (0.3 g), carnosine (0.3 g) and xylitol (21 g) have been added. The so obtained mixture has been subjected to stirring for 3 hours until the hyaluronate component was completely swollen. A silver-hyaluronate gel has thus been obtained having the following quali-quantitative (excluding water) composition (% in weight): Ag, 0.06%; sodium hyaluronate, 0.90%; amino acids 0.27%.

In the hyaluronic acid-based composition according to the invention, as disclosed in the Example 3, the L-proline and isoleucine amino acids, as well as the carnosine dipeptide, are used as cicatrizing agents, whilst the xylitol is used as a non-cariogenic sweetening agent. Therefore, the composition of Example 3 is usable effectively as an antimicrobial gel for oral topical use (see following Example 8). The percentages mentioned in the Example 3 can be varied, for example by reducing the ionic silver to 0.01%, increasing the sodium hyaluronate up to 1.6% and increasing the amino acids up to 0.54%. Moreover, as a person skilled in the art will understand, it is possible to use other amino acids instead of those cited above.

It should nevertheless be noted that the only essential components of the composition are the silver complex and the sodium hyaluronate, whilst amino acids and xylitol are optional excipients, namely non-essential components. Amino acids and xylitol can thus be eliminated from the formulation or be replaced with other known excipients, on the basis of the intended use of the composition according to the invention.

### Example 4 - Preparation of control hyaluronate gel

Sodium hyaluronate (3 g), L-proline (0.3 g), isoleucine (0.3 g), carnosine (0.3 g) and xylitol (21 g) have been added to 305.1 g of distilled water. The so obtained mixture has been subjected to stirring for 3 hours until the hyaluronate component was completely swollen. In this manner a control hyaluronate gel has been obtained, namely a gel containing sodium hyaluronate but free of the silver complexes according to the invention.

### Example 5 - Antimicrobial activity of Na⁺[Ag⁺-2-mercaptobenzoate²⁻] in aqueous solution

The experimental test disclosed below with reference to the complex Na⁺[Ag⁺-2-mercaptobenzoate²⁻] has been repeated on similar solutions of Na⁺[Ag⁺-3-mercaptobenzoate²⁻], Na⁺[Ag⁺-4-mercaptobenzoate²⁻] and Na⁺[Ag⁺⁻4-mercaptophenylacetate²⁻]. In all cases, an antimicrobial activity has been observed that is comparable among the different solutions.

The antimicrobial activity of aqueous solutions of Na⁺[Ag⁺-2-mercaptobenzoate²⁻] has been tested against the following strains of microorganisms (purchased from Diagnostic International Distribution S.p.A.): *Pseudomonas aeruginosa* ATCC 15442, *Staphylococcus aureus* ATCC 6538, *Escherichia coli* ATCC 10536, *Enterococcus hirae* ATCC 10541, *Candida albicans* ATCC 10231. Two mixtures of the different microorganisms have been prepared having concentrations expressed in colony forming units (CFUs) comprised between 1.5 × 10⁶ - 5.5 × 10⁶ and 1.5 × 10⁹ - 5.5 × 10⁹ for each species. 100 µl of the two mixtures have been inoculated in two Petri dishes containing TSA (Tryptone Soya Agar) solid culture medium. The inoculation has been carried out according to a known and normed analytic method, namely by depositing the liquid sample on the surface of the agar through micropipette and distributing the liquid sample on the surface of the agar by using sterile glass beads. Subsequently, in a central zone of the agar of each of the two Petri dishes 100 µl of aqueous solution of Na⁺[Ag⁺⁻2-mercaptobenzoate²⁻] (prepared as disclosed in the Example 1 and containing 0.19 % in weight of Ag⁺ ions) have been deposited. The two Petri dishes have been then incubated at 37° C for 24 h.

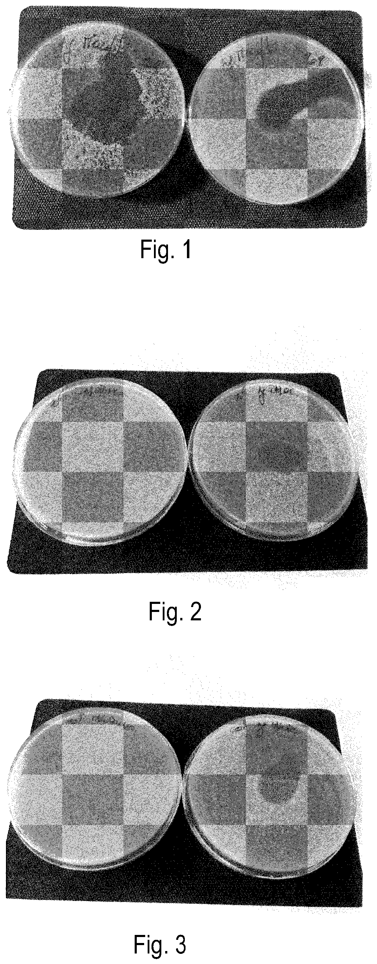

After the incubation, the capsules have been examined in order to evaluate the microbial proliferation (formation of colonies) and the size of the inhibition halo (namely, the size of the portion of medium in which the microbial proliferation has been inhibited) surrounding the agar zone on which the sample of Na⁺[Ag⁺-2-mercaptobenzoate²⁻] was deposited. As shown in Figure 1, in both Petri dishes two clear inhibition zones have been observed surrounding the depositing zones of the complex according to the invention. In the dish on the right, the microbial mixture had been inoculated having 1.5 × 10⁹ - 5.5 × 10⁹ CFU concentration and in the dish on the left the microbial mixture had been inoculated having concentration of 1.5 × 10⁶ - 5.5 × 10⁶ CFU. The obtained analytical result points out that the solutions containing complexed silver ions are able to inhibit the growth of 10⁸ CFU of gram-positive bacteria, gram-negative bacteria and of the *Candida albicans* fungin species.

### Example 6 - Antimicrobial activity of silver-hyaluronate gel

The antibacterial activity of a hyaluronic acid-based composition according to the invention, namely a hyaluronate gel containing Na⁺[Ag⁺-2-mercaptobenzoate²⁻] and of the corresponding control hyaluronate gel (containing sodium hyaluronate without the silver complex according to the invention) have been tested against the same pool of microorganisms (bacteria and fungi) disclosed in the Example 5.

Two mixtures of the different microorganisms have been prepared, having concentrations comprised between 1.5 × 10⁶ - 5.5 × 10⁶ CFU and 1.5 × 10⁹ - 5.5 × 10⁹ CFU for each species. Two 100 µl aliquots of each mixture have been inoculated separately (using the known method cited in the Example 5) in two Petri dishes containing TSA (Tryptone Soya Agar) solid culture medium, so as to obtain four Petri dishes altogether, namely 2 Petri dishes with microbial concentration of 1.5 × 10⁶ - 5.5 × 10⁶ CFU and two Petri dishes with microbial concentration of 1.5 × 10⁹ - 5.5 × 10⁹ CFU. On the two Petri dishes with microbial concentration of 1.5 × 10⁶ - 5.5 × 10⁶ CFU 100 µl of silver-hyaluronate gel of Example 3 (on the one dish) and 100 µl of control hyaluronate gel of Example 4 (on the other dish) have been deposited. Similarly, on the two Petri dishes with microbial concentration of 1.5 × 10⁹ - 5.5 × 10⁹ CFU 100 µl of silver-hyaluronate gel of the Example 3 (on the one dish) and 100 µl of control hyaluronate gel of the Example 4 (on the other dish) have been deposited. The four Petri dishes have then been incubated at 37°C for 24 h.

After the incubation, the capsules have been examined in order to evaluate the microbial proliferation and the size of the inhibition halo surrounding the agar zone on which the sample of silver-hyaluronate gel or the sample of control hyaluronate gel had been deposited. As shown in the Figures 2 and 3, clear inhibition zones have been observed surrounding the depositing zones of the silver-hyaluronate gel, namely only in the Petri dishes on which the hyaluronic acid-based composition according to the invention had been deposited. Both in the Figure 2 and in the Figure 3, the Petri dishes on which the sample of silver-hyaluronate gel has been deposited are positioned on the right. In the Figure 2 the Petri dishes with microbial concentration of 1.5 × 10⁶ - 5.5 × 10⁶ CFU are shown and in the Figure 3 the Petri dishes with microbial concentration of 1.5 × 10⁹ - 5.5 × 10⁹ CFU are shown. The obtained analytical result points out that the silver-hyaluronate gel is able to inhibit the growth of 10⁸ CFU of gram-positive bacteria, gram-negative bacteria and of the *Candida albicans* fungin species. On the other hand, even in the presence of reduced concentrations of microorganisms no antimicrobial activity of the control hyaluronate gel has been observed.

### Example 7 - Antimicrobial activity of the silver-hyaluronate gel against antibiotic-resistant bacteria.

The ability of a silver-hyaluronate gel according to the invention to destroy bacteria involved in the periodontal diseases has been tested. In particular, the antimicrobial activity has been evaluated of a hyaluronate gel containing Na⁺[Ag⁺-2-mercaptobenzoate²⁻] against bacteria of the genetically modified species *Tannerella forsythia* (TF) and *Porphyromonas gingivalis* (PG), purchased from Eurofins Genomics (MWG Operon, Ebersberg, Germany). In the plasmid of the aforesaid genetically modified species, target sequences of synthetic DNA are contained that give antibiotic-resistance. The bacteria have been cultured in a lysogeny broth (LB) containing kanamycin and ampicillin (50 µg /ml of broth) at 37°C for 24 h in a stirrer-incubator. Six 50 µl aliquots of microbial suspension (three aliquots of TF and three aliquots of PG), having 1 × 10¹⁰ - 5 × 10¹⁰ CFU/ml concentration, have been inoculated (using the known method cited in the Example 5) in six Petri dishes containing TSA (Tryptone Soya Agar) solid culture medium. In the central zone of medium (agar) of the Petri dishes containing TF three corresponding 50 µl samples have been deposited of: silver-hyaluronate gel containing Na⁺[Ag⁺-2-mercaptobenzoate²⁻] and sodium hyaluronate with molecular weight of 800-900 kDa; silver-hyaluronate gel containing Na⁺[Ag⁺-2-mercaptobenzoate²⁻] and sodium hyaluronate with molecular weight of 1500-2000 kDa; control hyaluronate gel containing sodium hyaluronate with molecular weight of 1500-2000 KDa. The same procedure has been applied for the three Petri dishes containing PG. After depositing the samples of gel, the six Petri dishes have been incubated at 37° C for 24 h.

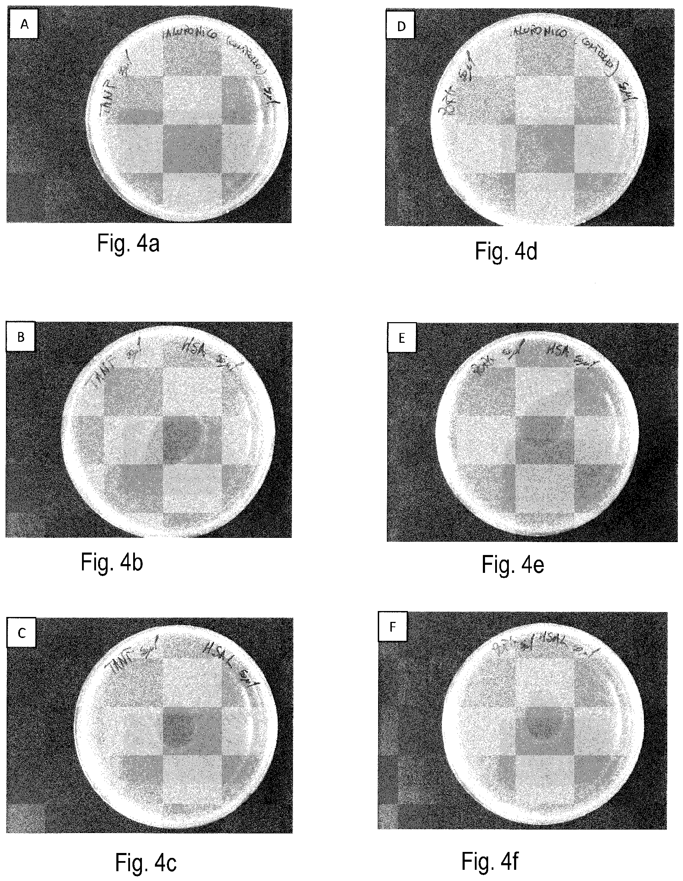

After the incubation, the capsules have been examined in order to evaluate the microbial proliferation and the size of the inhibition halo surrounding the agar zones on which the samples of silver-hyaluronate gel or the samples of control hyaluronate gel had been deposited. As shown in the Figures 4a to 4f, clear inhibition zones have been observed surrounding the depositing zones of the silver-hyaluronate gels according to the invention, regardless of the molecular weight of the sodium hyaluronate, whilst no antimicrobial activity (absence of inhibition halos) has been observed for the control hyaluronate gel.

The Figures 4a and 4d show the Petri dishes, marked by the letters A and D and containing respectively TF and PG, on which the samples of control hyaluronate gel have been deposited. The Figures 4b and 4e show the Petri dishes, marked by the letters B and E and containing respectively TF and PG, on which the samples of silver-hyaluronate gel according to the invention (Na⁺[Ag⁺-2-mercaptobenzoate²⁻] and sodium hyaluronate with molecular weight of 1500-2000 kDa) have been deposited. The Figures 4c and 4f show the Petri dishes, marked by the letters C and F and containing respectively TF and PG, on which the samples of silver-hyaluronate gel according to the invention (Na⁺[Ag⁺-2-mercaptobenzoate²⁻] and sodium hyaluronate with molecular weight of 800-900 kDa) have been deposited. The obtained analytical result shows the ability of the gel of sodium hyaluronate according to the invention to inhibit the growth of the bacterial species *Tannerella forsythia* and *Porphyromonas gingivalis* in concentrations of 10¹⁰ CFU/ml.

The antimicrobial activity of the silver-hyaluronate gel according to the invention has been also evaluated against a mixed microbial pool containing *Tannerella forsythia, Porphyromonas gingivalis, Pseudomonas aeruginosa, Staphylococcus aureus, Escherichia coli, Enterococcus hirae* and *Candida albicans* in concentrations in the order of 10¹⁰ CFU/ml. The aforesaid mixed microbial pool has been tested with samples of gel containing sodium hyaluronate and the silver complexes according to the invention (Na⁺[Ag⁺-2-mercaptobenzoate²⁻], Na⁺[Ag⁺-3-mercaptobenzoate²⁻], Na⁺[Ag⁺-4-mercaptobenzoate²⁻] and Na⁺[Ag⁺-4-mercaptophenylacetate²⁻]). Except for the gel containing Na⁺[Ag⁺-4-mercaptophenylacetate²⁻], which has shown a lesser antimicrobial activity, in the other cases well defined inhibition halos have been observed. The obtained analytical result shows the ability of the gel of sodium hyaluronate according to the invention to inhibit the growth of a mixed microbial pool in concentrations of 10¹⁰ CFU/ml.

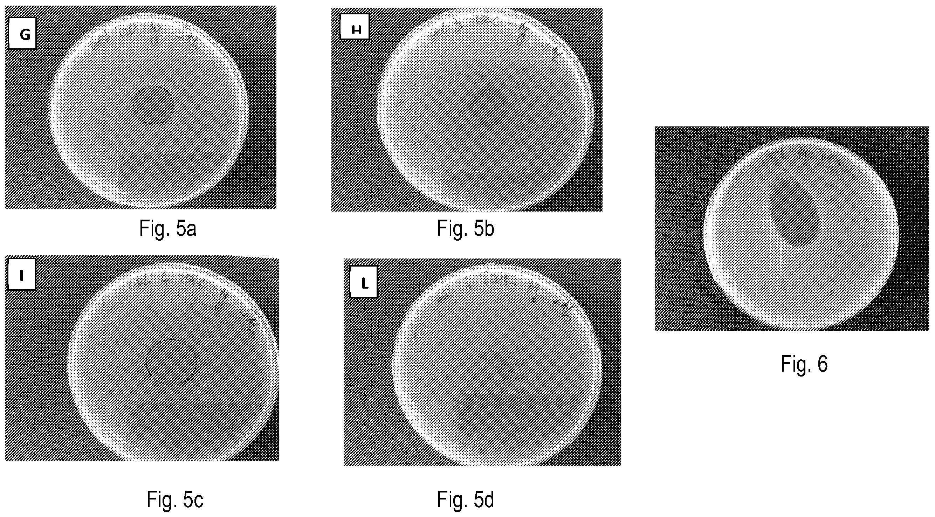

The Figures 5a to 5d show four Petri dishes (marked by the letters G, H, I, L) containing TSA (Tryptone Soya Agar) solid culture medium, in each of which a 50 µl aliquot has been inoculated (by using the known method disclosed in the Example 5) of a suspension containing *Tannerella forsythia, Porphyromonas gingivalis, Pseudomonas aeruginosa, Staphylococcus aureus, Escherichia coli, Enterococcus hirae* and *Candida albicans* in concentrations in the order of 10¹⁰ CFU/ml. In the central zone of medium (agar) of the four Petri dishes four corresponding 50 µl samples have been deposited of: silver-hyaluronate gel containing Na⁺[Ag⁺-2-mercaptobenzoate²⁻] and sodium hyaluronate with molecular weight of 1500-2000 kDa (Figure 5a); silver-hyaluronate gel containing Na⁺[Ag⁺-3-mercaptobenzoate²⁻] and sodium hyaluronate with molecular weight of 1500-2000 kDa (Figure 5b); silver-hyaluronate gel containing Na⁺[Ag⁺-4-mercaptobenzoate²⁻] and sodium hyaluronate with molecular weight of 1500-2000 kDa (Figure 5c); silver-hyaluronate gel containing Na⁺[Ag⁺-4-mercaptophenylacetate²⁻] and sodium hyaluronate with molecular weight of 1500-2000 kDa (Figure 5d).

### Example 8 - Antimicrobial activity in vivo of a silver-hyaluronate gel containing the complex Na⁺[Ag⁺ -2-mercaptobenzoate²⁻]

Six volunteers have been selected randomly, namely six patents with diagnosis of chronic periodontal disease (periodontitis). The selected patients (aged between 35 and 55) had not previously received any surgical or non-surgical periodontal therapy. None of the aforesaid patients was pregnant, had taken antibiotics, had used antibacterial mouthwash in the previous six months, had teeth with furcation involvement or had a history of alcohol and drug abuse. Before any treatment, at the initial time (day) T0, each of the six patients has been subjected to a subgingival microbiological sampling through the use of four paper points, which have been inserted into the periodontal pockets in four quadrants (right maxillary quadrant, left maxillary quadrant, right mandibular quadrant, left mandibular quadrant) and left therein for 20 seconds. The site of insertion of each paper point has been isolated by using cylindrical cotton swabs. Instructions have been given to all the patients to clean their teeth by applying 3 times a day a silver-hyaluronate gel according to the invention, containing sodium hyaluronate with molecular weight of 1500-2000 KDa and Na⁺[Ag⁺-2-mercaptobenzoate²⁻]. At the time T1, namely 10 days after the initial day T0, the six patients have been recalled to perform a second subgingival microbiological sampling in the same periodontal pockets examined at the initial time T0. The second subgingival microbiological sampling has been carried out with the same procedure used at the initial time T0. The paper points used for each patient at the time T0 and at the time T1 have been transferred in sterile test tubes and sent to a microbiological laboratory, for subsequent extraction of the DNA and PCR analysis (polymerase chain reaction). The bacterial count through PCR has been directed mainly at *Tannerella forsythia* (TF), *Treponema denticola* (TD), *Fusobacterium nucleatum* (FN) and *Campylobacter rectus* (CR), which are the bacterial species that are most involved in the periodontitis. The total bacterial load (TBL) has also been determined by PCR.

In the PCR analysis, the primers and the oligonucleotide probes were based on rRNA 16S gene sequences of the database of the human oral microbiome (HOMD 16S rRNA RefSeq Version 10.1), which counts 845 entries. Absolute quantification assays have been performed through PCR by using the 7500 Sequence Detection System (Applied Biosystems). The amplification profile has been initiated with an incubation period of 10 min at 95° C to activate polymerase, followed by an amplification in two steps of 15 sec at 95°C and 60 sec at 57°C for 40 cycles. All these tests have been performed by including controls without amplification probes to exclude contamination of the reagents. Plasmids containing specific DNA sequences (purchased from Eurofins MWG Operon, Ebersberg, Germany) have been used for the quantitative evaluation. These positive controls have been used to plot standard curves (reporting in orthogonal Cartesian axes the threshold cycle values versus the logarithm of the number of copies), which have been used to verify the amplification efficiency and for the quantification of targets in each sample (J Biol Regul Homeost Agents, 2017, 31(1): 257-262; J Biol. Regul. Homeost. Agents, 2016, 30(2 Suppl 1): 87-97; J Biol Regul Homeost Agents, 2015, 29(3 Suppl 1): 101-10.).

After the local treatment with the gel, namely at the time T1, a significant reduction in the quantity of bacteria has been observed. The absolute quantities of different bacterial species detected through bacterial count in periodontal pockets of patients affected by periodontitis, at the initial time T0 and at the time T1 (at 10 days from T0), are shown in the following Table 1:

**Table 1**

| PT | T0 | | | | | T1 | | | | |
|---|---|---|---|---|---|---|---|---|---|---|
| | TF1 | TD1 | FN1 | CR1 | TBL1 | TF2 | TD2 | FN2 | CR2 | TBL2 |
| 1 | 0 | 0 | 9867 | 1100 | 79600 | 0 | 0 | 5392 | 771 | 7589 |
| 2 | 1126 | 366 | 23724 | 6123 | 154660 | 0 | 0 | 1756 | 1381 | 5537 |
| 3 | 114 | 412 | 14592 | 2170 | 20568 | 0 | 0 | 1346 | 795 | 6909 |
| 4 | 1175 | 433 | 17785 | 1296 | 83520 | 224 | 91 | 5756 | 1656 | 8511 |
| 5 | 0 | 0 | 5785 | 1634 | 15068 | 0 | 0 | 3680 | 1114 | 5478 |
| 6 | 0 | 0 | 2007 | 988 | 10050 | 0 | 0 | 2140 | 730 | 5830 |

| | | | | | | | | | | |
|---|---|---|---|---|---|---|---|---|---|---|
| (T0: initial time (day) T1: 10 days from the initial time T0; PT: patient TF: *Tannerella forsythia;* TD: *Treponema denticola;* FN: *Fusobacterium nucleatum;* CR: *Campylobacter rectus;* TBL: total bacterial load). | | | | | | | | | | |

As shown in the Table 1, the average reduction for each bacterial species *(Tannerella forsythia, Treponema denticola, Fusobacterium nucleatum, Campylobacter rectus)* has been 90% as for the total bacterial load. Moreover, no side effects or adverse reactions have been observed (immediately or after a time lapse) following local application of the silver-hyaluronate gel according to the invention. The patients did not in fact report sensations of pain, burning, tingling and/or numbness during the entire treatment period, further stating that the applied gel was flavourless and odourless.

### Example 9 - Preparation of the Na₂⁺[Ag⁺-2-mercaptobenzoate²⁻-2-hydroxybenzoate²⁻] complex

0.2 g of 2-mercaptobenzoic acid (Aldrich, CAS: 4869-59-4; molecular weight: 154.19 g) have been suspended in 82.68 g of distilled water. To the so obtained suspension 2.6 ml of NaOH 1M (corresponding to two equivalents) have been added to solubilize the ligand, neutralizing the two protons bonded to the sulphur and to the carboxyl function. After complete dissolution of the ligand, which dissolution has occurred in a time comprised between 20 and 30 min, the pH was measured and adjusted at 7. 0.22 g of AgNO₃ (Aldrich, CAS: 7761-88-8; molecular weight: 169.87) dissolved in 63.81 g of distilled water have been added, corresponding to a 1:1 stechiometric ratio to the ligand. The reaction is almost instantaneous and leads to the formation of an anionic complex, in which the Ag+ ion is bonded to the negative sulphurous component of the 2-mercaptobenzoate. After a few minutes a solution has been added containing 0.18 g of 2-hydroxybenzoic acid, or salicylic acid (CAS: 69-72-7; molecular weight 138.12 g), solubilized in 1.29 ml of NaOH 1M and 45.82 g of distilled water. The silver ion is thus complexed by both the 2-mercaptobenzoate ligand and the 2-hydroxybenzoate ligand. The solution has been concentrated in rotating evaporator and the complex has been precipitated through addition of acetone, separated by filtration and dried at 60°C. The complex has been characterized through elemental analysis CHNS and determination of Ag. For the elemental analysis of carbon, hydrogen and sulphur, a LECO CHNS analyzer has been used. The silver has been determined with an optical plasma atomic emission spectrophotometer Optima 3100 XL (Perkin Elmer). Calculated percentages of the elements for Na₂AgC₁₄H₉O₅S: C, 37.95%; H, 2.05%; S, 7.24; Ag 24.34. Found percentages of the elements for Na₂AgCₗ₄H₉O_{S}S: C, 38.02%; H, 2.18%; S, 7.90; Ag, 25.82.

### Example 10 - Preparation of silver-hyaluronate gel containing the complex Na₂⁺[Ag⁺-2-mercaptobenzoate²⁻-2-hvdroxybenzoate²⁻]

0.2 g of 2-mercaptobenzoic acid have been suspended in 82.68 g of distilled water and 2.6 ml of NaOH 1M have been added. After complete dissolution of the ligand, the pH has been adjusted at 7. 0.22 g of AgNO₃ dissolved in 63.81 g of distilled water have been then added and, after a few minutes, a solution has been added containing 0.18 g of 2-hydroxybenzoic acid solubilized in 1.29 ml of NaOH 1M and 45.82 g of distilled water. After 30 minutes of stirring, 0.2g of carnosine, 2 g of sodium hyaluronate and 1 g of NATROSOL (hydroxyethyl cellulose) have been added. The suspension has been kept under continuous stirring for another 6 hours, until complete gelification.

### Example 11 - Antimicrobial activity of silver-hyaluronate gel containing the complex Na₂⁺[Ag²⁺-2-mercaptobenzoate²⁻-2-hydroxybenzoate²⁻]

The antimicrobial activity of the gel containing Na₂⁺[Ag⁺-2-mercaptobenzoate²⁻-2-hydroxybenzoate²⁻] has been tested against the following strains of microorganisms (purchased from Diagnostic International Distribution S.p.A.): *Pseudomonas aeruginosa* ATCC 15442, *Staphylococcus aureus* ATCC 6538, *Escherichia coli* ATCC 10536, *Enterococcus hirae* ATCC 10541, *Candida albicans* ATCC 10231. A mixture of the different strains of microorganisms has been prepared, having concentrations expressed in colony-forming units (CFU) comprised between 1 × 10¹⁰ and 5 × 10¹⁰. 100 µl of the mixture have been inoculated in a Petri dish containing TSA (Tryptone Soya Agar) solid culture medium. The inoculation has been carried out according to a known and normed analytic method, namely by depositing the liquid sample on the surface of the agar through micropipette and distributing the liquid sample on the surface of the agar by using sterile glass beads. Subsequently, 100 µl of gel have been deposited in a central zone of the agar in the Petri dish. After the incubation, as shown in Figure 6, in the solid medium contained in the Petri dish a clear inhibition halo of the microbial growth formed. This shows that the gel based on hyaluronic acid and containing the complex Na₂⁺[Ag⁺⁻2-mercaptobenzoate²⁻-2-hydroxybenzoate²⁻] is able to inhibit the growth of 10⁹ CFU of gram-positive bacteria, gram-negative bacteria and of the *Candida albicans* fungin species.

As a person skilled in the art will understand, variations on and/or additions to what has been disclosed above are possible. For example, although the previously disclosed Examples refer to preparations of complexes or gels on laboratory scale, the person skilled in the art is able to modify appropriately the procedures of preparation of the Examples so as to make these procedures suitable for a production on industry scale. In particular, the person skilled in the art is able to modify the doses of the substances appropriately and/or use apparatuses that are different from those disclosed in the Examples.

Moreover, although the Example 8 refers specifically to the use of a silver-hyaluronate composition (gel) according to the invention in the treatment of periodontal diseases, the person skilled in the art has the necessary knowledges for the use of the same composition for the other previously mentioned applications, namely antimicrobial fillers in cosmetic surgery, intra-articular orthopaedic treatments (for example, viscosupplementation), adjuvant for promoting the healing of wounds, possibly by modifying appropriately the composition according to the invention.

## Claims

1. Bi-coordinated silver (I) complex, having antimicrobial activity and the general formula:
Na⁺2 [Ag⁺(L²⁻)(M²⁻)]
wherein L is a ligand consisting of 2-mercaptobenzoic acid and M is a ligand different from the said L ligand and consisting of 2-hydroxybenzoic acid, said complex being incorporable into a hyaluronic acid composition in a stable manner.

2. Hyaluronic acid composition containing the bi-coordinated silver (I) complex according to claim 1 for use as medicament having antimicrobial activity.

3. Hyaluronic acid composition containing a mono-coordinated silver (I) complex for use as medicament having antimicrobial activity, said mono-coordinated silver (I) complex having antimicrobial activity and general formula:
Na⁺[Ag⁺(L²⁻)]
wherein L is a ligand selected from the group consisting of: 4-mercaptobenzoic acid, 3-mercaptobenzoic acid, 2-mercaptobenzoic acid and 4-mercaptophenylacetic acid.

4. Hyaluronic acid composition according to claim 2, or 3, for use in the treatment of periodontal diseases.

5. Hyaluronic acid composition according to claim 2, or 3, for use as antimicrobial filler in cosmetic surgery.

6. Hyaluronic acid composition according to claim 2, or 3, for use in intra-articular orthopaedic treatments.

7. Hyaluronic acid composition according to claim 2, or 3, for use as adjuvant for promoting the healing of wounds.

8. Hyaluronic acid composition for use according to any one of claims 2 to 7, wherein said hyaluronic acid is present in the form of a physiologically and pharmaceutically acceptable salt thereof, said salt being sodium hyaluronate.

9. Composition for use according to claim 8, in which said sodium hyaluronate has a molecular weight comprised between 800 KDa and 2000 KDa.

10. Composition for use according to claim 8, or 9, containing from 0.90% in weight to 1.6% in weight of said sodium hyaluronate.

11. Composition for use according to any one of claims 2 to 10, containing from 0.01% in weight to 0.06% in weight of ionic silver.

12. Composition for use according to any one of claims 2 to 11, optionally comprising one or more excipients selected from the group consisting of: amino acids, dipeptides and xylitol.

## Patentansprüche

1. Zweifach koordinierter Silber-(I)-Komplex, der eine antimikrobielle Wirkung und die allgemeinen Formel aufweist:
Na⁺₂ [Ag⁻(L²⁻)(M²⁻)],
wobei L ein Ligand ist, der aus 2-Mercaptobenzoesäure besteht, und M ein Ligand ist, der sich von dem L-Liganden unterscheidet und aus 2-Hydroxybenzoesäure besteht, wobei der Komplex auf stabile Weise in eine Hyaluronsäure-Zusammensetzung einbaubar ist.

2. Hyaluronsäure-Zusammensetzung, die den zweifach koordinierten Silber-(I)-Komplex gemäß Anspruch 1 enthält, zur Verwendung als Medikament, das antimikrobieller Aktivität aufweist.

3. Hyaluronsäure-Zusammensetzung, die einen einfach koordinierten Silber-(I)-Komplex enthält, zur Verwendung als Medikament mit antimikrobieller Aktivität, wobei der einfach koordinierte Silber-(I)-Komplex antimikrobielle Aktivität und die allgemeine Formel aufweist:
Na⁺[Ag⁺(L²⁻)],
wobei L ein Ligand ist, der ausgewählt ist aus der Gruppe bestehend aus: 4-Mercaptobenzoesäure, 3-Mercaptobenzoesäure, 2-Mercaptobenzoesäure und 4-Mercaptophenylessigsäu re.

4. Hyaluronsäure-Zusammensetzung gemäß Anspruch 2 oder 3 zur Verwendung bei der Behandlung von parodontalen Erkrankungen.

5. Hyaluronsäure-Zusammensetzung gemäß Anspruch 2 oder 3 zur Verwendung als antimikrobieller Füllstoff in der kosmetischen Chirurgie.

6. Hyaluronsäure-Zusammensetzung gemäß Anspruch 2 oder 3 zur Verwendung bei intraartikulären orthopädischen Behandlungen.

7. Hyaluronsäure-Zusammensetzung gemäß Anspruch 2 oder 3 zur Verwendung als Adjuvans zur Förderung der Heilung von Wunden.

8. Hyaluronsäure-Zusammensetzung zur Verwendung gemäß einem der Ansprüche 2 bis 7, wobei die Hyaluronsäure in Form eines physiologisch und pharmazeutisch akzeptablen Salzes davon vorliegt, wobei das Salz Natriumhyaluronat ist.

9. Zusammensetzung zur Verwendung gemäß Anspruch 8, bei der das Natriumhyaluronat ein Molekulargewicht zwischen 800 KDa und 2000 KDa aufweist.

10. Zusammensetzung zur Verwendung gemäß Anspruch 8 oder 9, die 0,90 Gew.-% bis 1,6 Gew.-% des Natriumhyaluronats enthält.

11. Zusammensetzung zur Verwendung gemäß einem der Ansprüche 2 bis 10, die 0,01 Gew.-% bis 0,06 Gew.-% ionisches Silber enthält.

12. Zusammensetzung zur Verwendung gemäß einem der Ansprüche 2 bis 11, die optional einen oder mehrere Hilfsstoffe enthält, die ausgewählt sind aus der Gruppe bestehend aus: Aminosäuren, Dipeptiden und Xylitol.

## Revendications

1. Complexe d'argent (I) bi-coordonné, ayant une activité antimicrobienne et la formule générale :
Na⁺₂[Ag⁺(L²⁻)(M²⁻)]
dans laquelle L est un ligand consistant en acide 2-mercaptobenzoïque et M est un ligand différent dudit ligand L et consistant en acide 2-hydroxybenzoïque, ledit complexe étant incorporable dans une composition d'acide hyaluronique d'une manière stable.

2. Composition d'acide hyaluronique contenant le complexe d'argent (I) bi-coordonné selon la revendication 1, pour une utilisation en tant que médicament ayant une activité antimicrobienne.

3. Composition d'acide hyaluronique contenant un complexe d'argent (I) mono-coordonné pour une utilisation en tant que médicament ayant une activité antimicrobienne, ledit complexe d'argent (I) mono-coordonné ayant une activité antimicrobienne et la formule générale :
Na⁺[Ag⁺(L²⁻)]
dans laquelle L est un ligand choisi dans le groupe constitué par : l'acide 4-mercaptobenzoïque, l'acide 3-mercaptobenzoïque, l'acide 2-mercaptobenzoïque et l'acide 4-mercaptophénylacétique.

4. Composition d'acide hyaluronique selon la revendication 2 ou 3, pour une utilisation dans le traitement de maladies parodontales.

5. Composition d'acide hyaluronique selon la revendication 2 ou 3, pour une utilisation en tant que charge antimicrobienne en chirurgie plastique.

6. Composition d'acide hyaluronique selon la revendication 2 ou 3, pour une utilisation dans des traitements orthopédiques intra-articulaires.

7. Composition d'acide hyaluronique selon la revendication 2 ou 3, pour une utilisation en tant qu'adjuvant pour promouvoir la cicatrisation de plaies.

8. Composition d'acide hyaluronique pour une utilisation selon l'une quelconque des revendications 2 à 7, dans laquelle ledit acide hyaluronique est présent sous la forme d'un sel physiologiquement et pharmaceutiquement acceptable de celui-ci, ledit sel étant le hyaluronate de sodium.

9. Composition pour une utilisation selon la revendication 8, dans laquelle ledit hyaluronate de sodium a une masse moléculaire comprise entre 800 kDa et 2 000 kDa.

10. Composition pour une utilisation selon la revendication 8 ou 9, contenant de 0,90 % en poids à 1,6 % en poids dudit hyaluronate de sodium.

11. Composition pour une utilisation selon l'une quelconque des revendications 2 à 10, contenant de 0,01 % en poids à 0,06 % en poids d'argent ionique.

12. Composition pour une utilisation selon l'une quelconque des revendications 2 à 11, comprenant optionnellement un ou plusieurs excipients choisis dans le groupe constitué par : les acides aminés, les dipeptides et le xylitol.
